# EUROPÄISCHE PATENTANMELDUNG

(11) **EP 1 961 454 A2**
(43) Veröffentlichungstag der Anmeldung: **27.08.2008**
(21) Anmeldenummer: 08001862.5
(22) Anmeldetag: 01.02.2008
(51) Int. Cl.: A61Q 19/00, A61Q 19/08, A61Q 19/10, A61K 8/96, A61K 8/97

(54) **Mittel zur Körper- oder Schönheitspflege**

(30) Priorität: 24.02.2007 DE 102007009125
(71) Anmelder: ADA Cosmetic GmbH, 77694 Kehl/Baden (DE)
(72) Erfinder: Raatz, Claudia, 79365 Rheinhausen (DE)
(74) Vertreter: Maucher, Wolfgang

(57) **Zusammenfassung**

Die Erfindung betrifft ein Mittel zur Körper- oder Schönheitspflege, dass zur äußeren Anwendung bestimmt ist. Für das erfindungsgemäße Körper- oder Schönheitspflegemittel ist kennzeichnend, dass es Würenloser Heilgestein enthält und zum Aufbringen auf die Haut als Creme oder Emulsion oder als Tensid ausgebildet ist. Würenloser Heilgestein wird bislang nur als Pulver angeboten, das entweder innerlich angewendet, in Form eines Wickels aufgebraucht oder zu einem Vollbad zugeführt werden kann. Diese Darreichungsformen erschweren eine breite und jederzeitige Anwendung des Heilgesteins. Das erfindungsgemäße Körper- oder Schönheitspflegemittel enthält Würenloser Heilgestein, dass darin eine entzündungshemmende, vitalisierende, energetisierende, wundheilungsfördernde, juckreizstillende und bindegewebsstärkende Wirkung entfaltet. Da das erfindungsgemäße Körper- oder Schönheitspflegemittel als eine auf die Haut aufbringbare Creme oder Emulsion dargeboten wird, steht die erfindungsgemäße Körper- oder Schönheitspflegecreme jederzeit zur Verfügung, ohne dass beispielsweise aufwendige Wickel angefertigt und bereit gestellt werden müssten.

## Beschreibung

Die Erfindung betrifft ein Mittel zur Körper- oder Schönheitspflege, das zur äußeren Anwendung bestimmt ist.

Körper- oder Schönheitspflegemittel sind bereits in den verschiedensten Zusammensetzungen und Darreichungsformen bekannt. So kennt man beispielsweise auch mineralstoffhaltige Körper- oder Schönheitspflegemittel, die zur inneren Anwendung als Nahrungsergänzungsmittel angeboten werden.

Für das erfindungsgemäße Körper- oder Schönheitspflegemittel, das zur äußeren Anwendung bestimmt ist, ist kennzeichnend, dass das Körper- oder Schönheitspflegemittel Würenloser Heilgestein enthält und zum Aufbringen auf die Haut als Creme oder Emulsion oder als Tensid ausgebildet ist.

Würenloser Heilgestein ist ein Muschelkalksandstein, der in der Nähe von Zürich für therapeutische Zwecke abgebaut, gereinigt, verfeinert und zu mehlfeinem Pulver verarbeitet wird, um anschließend zur inneren Anwendung eingenommen oder zur äußeren Anwendung in Form eines Wickels eingesetzt zu werden. Würenloser Heilgestein wird eingesetzt bei sämtlichen Problemen mit dem Bewegungsapparat, z.B. rheumatischen Erkrankungen, Entzündungen, Sportverletzungen und Unfallfolgen. Darüber hinaus wurden auch positive Erfahrungen bei allergischen Reaktionen und Ekzemen verzeichnet. Würenloser Heilgestein enthält u.a. Kalziumkarbonat, Kieselsäure in Form von Quarz und anderen Verbindungen sowie tonartige Mineralien wie Illit, Montmorillonit, Eisen, Mangan, Magnesium, Kalium, Phosphat, Titan und Schwefel. Darüber hinaus zeichnet sich Würenloser Heilgestein durch eine hohe energetische Wirkung aus. Darüber hinaus wirkt sich dieses Heilgestein auch positiv bei einer Übersäuerung des Körpers aus.

Würenloser Heilgestein wird bislang nur als Pulver angeboten, das entweder innerlich angewendet, in Form eines Wickels aufgebracht oder zu einem Vollbad zugeführt werden kann. Diese Darreichungsformen erschweren eine breite und jederzeitige Anwendung des Heilgesteins.

Das erfindungsgemäße Körper- oder Schönheitspflegemittel enthält Würenloser Heilgestein, dass darin eine entzündungshemmende, vitalisierende, energietisierende, wundheilungsfördernde, juckreizstillende und - wegen seines hohen Siliciumanteils - auch Bindegewebes stärkende Wirkung entfaltet. Da das erfindungsgemäße Körper- oder Schönheitspflegemittel als eine auf die Haut aufbringbare Creme oder Emulsion dargeboten wird, steht die erfindungsgemäße Körper- oder Schönheitspflegecreme jederzeit zur Verfügung, ohne dass beispielsweise aufwendige Wickel angefertigt und bereitgestellt werden müssten. Das erfindungsgemäße Körper- oder Schönheitspflegemittel ist auch als Peeling-Produkt geeignet.

Die körper- und schönheitspflegende Wirkung des erfindungsgemäßen Körper- oder Schönheitspflegemittels wird noch begünstigt, wenn das Körper- oder Schönheitspflegemittel ein Extrakt von Ringelblume enthält.

Besonders vorteilhaft ist es, wenn das Körper- oder Schönheitspflegemittel ein Extrakt von zumindest einem Alpenkraut enthält.

Dabei sieht eine bevorzugte Ausführungsform gemäß der Erfindung, die sich durch eine besonders hohe körper- und schönheitspflegende Wirkung auszeichnet, vor, dass das Körper-oder Schönheitspflegemittel ein Extrakt von zumindest einem der nachfolgend genannten Alpenkräutern enthält: Edelweiß, Meisterwurz, weißer Andorn, Holunder, Spitzwegerich, und/oder Schmetterlingsstrauch.

Eine bevorzugte Weiterbildung gemäß der Erfindung sieht vor, dass das Körper- oder Schönheitspflegemittel zumindest einen Anti-Aging-Wirkstoff enthält. Ist das erfindungsgemäße Körper- oder Schönheitspflegemittel als kosmetisches Produkt ausgebildet, so kann es vorteilhaft sein, wenn das Körper-oder Schönheitspflegemittel als Anti-Aging-Wirkstoff zumindest eines der nachfolgend genannten Bestandteile enthält:

Aminosäuren und Derivate, Proteine und hydrolysierte Form, Kollagene und Derivate, Elastin und Derivate, Keratin und Derivate, Peptide, Vitamine und Derivate, Mineralien und Derivate, Spurenelemente, Hydroxysäuren und Derivate, Pflanzenextrakte und deren isolierte Wirkstoffe, Hyaluronsäure und Derivate, Pflanzenöle, Ceramide, Sphingolipide, Cerebroside, Lichtschutzfilter, Enzyme und Derivate, Coenzyme und Derivate, Wirkstoffe aus Plankton, Wirkstoffe aus Hefen, Wirkstoffe aus Algen, Wirkstoffe aus Pilzen, Wirkstoffe aus Tang, Wirkstoffe aus sonstigen Meerestieren und -pflanzen, biotechnologisch hergestellte Rohstoffe, Pyrrolidoncarbonsäure und Derivate, Bestandteile des NMF (Natural Moisturizing Factor), Cyclodextrin und Derivate, Chitosan und Derivate, Chitin und Derivate, Zucker und Zuckeralkohole und Derivate, Lecithin und Derivate, Chondroitinsulfat, Glucosaminsulfat, Wirkstoffe, die durch Fermentation aus Mikroorganismen hergestellt werden, Wirkstoffe aus Mikroorganismen, Carnithin, Ursolsäure, Siliciumdioxid und Derivate, Stärke und Derivate, Silikone und Derivate, Nylon und Derivate, Polyethylen und Derivate, Polysaccharide und Derivate, Antioxidantien und Derivate, ATP und Derivate, Glykogen und Derivate sowie Kreatin und Derivate.

## Patentansprüche

1. Mittel zur Körper- oder Schönheitspflege, dass zur äußeren Anwendung bestimmt ist, **dadurch gekennzeichnet, dass** das Körper- oder Schönheitspflegemittel Würenloser Heilgestein enthält und zum Aufbringen auf die Haut als Creme oder Emulsion oder als Tensid ausgebildet ist.

2. Körper- oder Schönheitspflegemittel nach Anspruch 1, **dadurch gekennzeichnet, dass** das Körper- oder Schönheitspflegemittel ein Extrakt von Ringelblume enthält.

3. Körper- oder Schönheitspflegemittel nach Anspruch 1 oder 2, **dadurch gekennzeichnet, dass** das Körper- oder Schönheitspflegemittel ein Extrakt von zumindest einem Alpenkraut enthält.

4. Körper- oder Schönheitspflegemittel nach Anspruch 3, **dadurch gekennzeichnet, dass** das Körper- oder Schönheitspflegemittel ein Extrakt von zumindest einem der nachfolgend genannten Alpenkräutern enthält: Edelweiß, Meisterwurz, weißer Andorn, Holunder, Spitzwegerich, Schmetterlingsstrauch.

5. Körper- oder Schönheitspflegemittel nach einem der Ansprüche 1 bis 4, **dadurch gekennzeichnet, dass** das Körper- oder Schönheitspflegemittel einen Anti-Aging-Wirkstoff enthält.
